# EUROPÄISCHE PATENTSCHRIFT

(11) **EP 1 423 060 B1**
(45) Veröffentlichungstag und Bekanntmachung des Hinweises auf die Patenterteilung: **20.12.2006**
(21) Anmeldenummer: 02797942.6
(22) Anmeldetag: 31.08.2002
(51) Int. Cl.: A61F 2/16

(54) **VERSTEIFBARER KAPSELSPANNRING**
RIGIDIFIABLE CAPSULE TENSIONING RING
BAGUE DE SERRAGE CAPSULAIRE RIGIDIFIABLE

(30) Priorität: 06.09.2001 DE 10143634
(43) Veröffentlichungstag der Anmeldung: 02.06.2004
(73) Patentinhaber: Preussner, Paul Rolf, Dr., 55131 Mainz (DE)
(72) Erfinder: Preussner, Paul Rolf, Dr., 55131 Mainz (DE)
(86) Internationale Anmeldenummer: PCT/EP2002/009755
(87) Internationale Veröffentlichungsnummer: WO 2003/022182

(56) Entgegenhaltungen:
- WO-A-97/49354
- DE-A- 19 530 465
- US-A- 4 575 373
- US-A- 5 725 575
- US-A1- 2001 004 708

## Beschreibung

Bei der Erfindung handelt es sich um ein Implantat, das im Rahmen der Operation der getrübten Augenlinse in den Kapselsack des menschlichen Auges eingesetzt werden kann. Ein Kapselspannring gemäß Oberbegriff von Patentanspruch 1 ist z.B. aus US 2001/0004708 bekannt.

### Stand der Technik

Bei einer Operation der getrübten Augenlinse (grauer Star, Katarakt) wird heute im allgemeinen der Kapselsack der Linse eröffnet, das trübe Linseninnere abgesaugt und anschließend eine Kunstlinse in den Kapselsack eingesetzt. Im Lauf der folgenden Wochen und Monate kommt es zu einer individuell mehr oder weniger ausgeprägten Schrumpfung des Kapselsacks. Diese ist vor allem dann unerwünscht, wenn zu einem späteren Zeitpunkt die Intraokularlinse gewechselt werden soll (z.B. bei Kindern), oder wenn eine möglichst weite Ausspannung des Kapselsacks auf Dauer aus anderen Gründen gewünscht wird, etwa bei torischen Intraokularlinsen oder bei einem akkommodativen Implantat, wie z.B. aus DE19904441 bekannt. Um eine Ausspannung des Kapselsacks zu gewährleisten, sind dem Stand der Technik entsprechende elastische, in ihrer Ringform unterbrochene, offene Ringe, sogenannte Kapselspannringe, bekannt. Diese können den Schrumpfungsprozeß des Kapselsacks zwar reduzieren, aber nicht völlig verhindern. Der Grund hierfür ist, daß der Kapselspannring einerseits nicht zu steif sein darf, damit er in die sehr fragile Kapsel noch eingeführt werden kann ohne sie zu verletzen, andererseits aber gerade deswegen dem Schrumpfungsprozeß keine ausreichende Gegenkraft entgegensetzen kann. Mechanische Elemente, mit denen man nach der Einführung in den Kapselsack eine Versteifung des Ringes bewirken könnte, wie z.B. Stifte, Niete, Rasten (z.B. DE4030899, DE19951148, US2001/0004708, DE19637692) etc. sind intraoperativ wegen ihrer Kleinheit, der Verletzlichkeit des Kapselsacks und der für ihre Handhabung zusätzlich benötigten Operationsdauer wenig geeignet.

**Aufgabe der vorliegenden Erfindung** ist es, einen Kapselspannring verfügbar zu machen, der nach seiner Einführung in den Kapselsack auf einfache Weise versteift werden kann.

### Kurze Darstellung der Erfindung

Die Aufgabe wird erfindungsgemäß entsprechend den Merkmalen des Anspruchs 1 gelöst. Der Kapselspannring ist so ausgeführt, daß sich seine offenen Enden nach der Implantation in einem Überlappbereich ohne Zwischenraum berühren. Der Überlappbereich muß innerhalb der nötigenfalls zu erweiternden Pupillaröffnung liegen, damit er von außen sichtbar ist.

Zumindest in diesem Berührungsbereich ist das Material so gefärbt, daß es das Licht eines CW-Lasers weitgehend absorbiert, für dessen Wellenlänge die brechenden Medien des Auges transparent sind. Mit Hilfe eines solchen Lasers können dann die Enden entsprechend den bekannten Prinzipien der Schweißtechnik im Inneren des Kapselsacks verschweißt werden, wenn die aneinander anliegenden Teile von ihrer Oberflächenform geeignet sind, ineinanderfließende Schmelzonen auszubilden.

### Bevorzugte Ausführung und Ausführungsalternativen

In seiner bevorzugten Ausführung besteht der Kapselspannring aus Polymethylmetacrylat, da sich dieses Material für Kapselspannringe wegen seiner mechanischen Eigenschaften und seiner Biokompatibilität bewährt hat, und weil es leicht verschweißt werden kann.
Der Außendurchmesser des Ringes liegt typisch bei etwa 12-14mm, damit er die Kapsel, die einen Äquatordurchmesser von ca. 9-11mm hat, federnd ausspannen kann. Die Materialstärke des Ringes liegt bei etwa 0.2mm. Die Enden des Ringes sind fahnenartig ca. 2.5mm so nach innen verbreitert, daß es nach der Implantation zu einer teilweisen Überlappung dieser Fahnen kommt, wobei sich, durch den üblichen Implantationsvorgang bedingt, das später ins Auge eingeführte Ende, im folgenden "zweites Ende" genannt, vor das zuerst eingeführte legt. "Vor" bzw. "vorne" bedeutet dabei hier und im folgenden in Richtung auf die Hornhaut des Auges, d.h. parallel zur optischen Achse, "innen" in Richtung Ring- bzw. Augenmitte, d.h. senkrecht zur optischen Achse. Die Fahnen sind an den sich berührenden Flächen so ausgeführt, daß sie im Grünen absorbieren, denn in diesem Wellenlängenbereich arbeiten die meisten in der Augenheilkunde verwendeten Laser (511nm bzw. 532nm). Die Einfärbung darf aber zumindest beim zweiten Fahnenende nicht durchgängig, sondern nur auf der Rückseite vorhanden sein, da das Laserlicht sonst nicht an der gewünschten Stelle absorbiert würde. Die fahnenartige Verbreiterung nach innen ist auch deswegen erforderlich, weil der Kapselspannring ansonsten nach der Implantation durch die Iris verdeckt wird und somit für das Laserlicht nicht mehr zugänglich ist.
In einer anderen Ausführung enthält die Fahne des zweiten Endes kleine, kegelförmige Bohrungen, wobei das Bohrungsende mit dem kleineren Durchmesser nach der Implantation der anderen Fahne anliegt. Die Verschweißung erfolgt dann im Inneren der Bohrungen. Vorteilhaft ist bei dieser Ausführung, daß das gesamte Material gleichmäßig absorbieren, d.h. homogen eingefärbt sein kann.
In einer weiteren Ausführung ragt die zweite Fahne etwas weniger weit nach innen als die erste. Die zweite Fahne ist zur ersten hin abgeschrägt. Dies ermöglicht eine gute Verschweißbarkeit im Bereich der Abschrägung. Auch bei dieser Ausführung kann das gesamte Material homogen eingefärbt sein.

## Patentansprüche

1. Kapselspannring zum Implantieren in den Kapselsack der menschlichen Augenlinse, ausgeführt als in seiner Ringstruktur unterbrochener und daher offener Ring, **gekennzeichnet dadurch,**
• **daß** die äußere Form des Ringes so ausgeführt ist, daß sich die offenen Enden nach der Implantation in einem Bereich berühren, der von außerhalb des Auges optisch zugänglich ist, und
• **daß** die Farbe des Materials im Berührungsbereich so gewählt ist, daß es das Licht eines CW-Lasers absorbiert, für den die brechenden Medien des Auges durchlässig sind, und
• **daß** im Berührungsbereich die Oberflächenform der aneinander anliegenden Teile so ausgeführt ist, daß sich beim Erhitzen eines umschriebenen Bereiches mit einem CW-Laser an beiden Enden ineinanderfließende Schmelzzonen ausbilden können.

2. Kapselspmillring nach Anspruch 1, **gekennzeichnet dadurch, daß** seine beiden Enden fahnenartig in Richtung Ringmitte verbreitert sind, so daß diese beiden Fahnen nach der Implantation übereinanderliegen, wobei, bezogen auf die Richtung zur Hornhaut, die Rückseite der vorderen und mindestens die Vorderseite der hinteren Fahne das Laserlicht absorbieren, während die vordere Fahne ansonsten dafür transparent ist.

3. Kapselspannring nach Anspruch 1, **gekennzeichnet dadurch, daß** seine beiden Enden fahnenartig in Richtung Ringmitte verbreitert sind, so daß diese beiden Fahnen nach der Implantation übereinanderliegen, wobei, bezogen auf die Richtung zur Hornhaut, die vordere Fahne kraterförmige Bohrungen aufweist, die sich in Richtung zur hinteren Fahne verjüngen.

4. Kapselspannring nach Anspruch 1, **gekennzeichnet dadurch, daß** seine beiden Enden fahnenartig in Richtung Ringmitte verbreitert sind, so daß diese beiden Fahnen nach der Implantation übereinanderliegen, wobei, bezogen auf die Richtung zur Hornhaut, die vordere Fahne weniger weit nach innen zur Ringmitte ragt als die hintere, und im Berührungsbereich zur hinteren Fahne hin abgeschrägt ist.

## Claims

1. Capsular tension ring for the impantation into the capsule of the lens of the human eye, constructed in the form of an interrupted and therefore open ring, **characterized by**
• an overall form, in which the open ends of the ring after implantation touch each other in an area which is optically accessible from outside the eye, and
• the color of the material in the touching area, which is chosen to absorb the light of a cw-laser, for which the refracting media of the eye are transparent, and
• the surface shape of the parts touching each other, which is manufactured in such a way in the touching area, that in case of heating up defined spots by a cw-laser, melting zones can occur in both ends which merge into one another.

2. Capsular tension ring according to claim 1, **characterized by** the fact that both ends are widened to the direction of the ring center in the form of flags, causing that these flags are lying upon each other after implantation, with, relative to the direction of the cornea, the back surface of the anterior and at least the anterior surface of the posterior flag absorb the laser light, whereas the anterior flag is, apart from that, transparent for this light.

3. Capsular tension ring according to claim 1, **characterized by** the fact that both ends are widened to the direction of the ring center in the form of flags, causing that these flags are lying upon each other after implantation, with, relative to the direction of the cornea, the anterior flag having drilled holes in the form of craters, narrowing to the direction of the posterior flag.

4. Capsular tension ring according to claim 1, **characterized by** the fact that both ends are widened to the direction of the ring center in the form of flags, causing that these flags are lying upon each other after implantation, with, relative to the direction of the cornea, the anterior flag pointing less far to the ring center than the posterior one, and with the anterior flag being bevelled to the posterior one in the touching area.

## Revendications

1. Anneau de tension capsulaire pour implantation dans le sac capsulaire du cristallin humain réalisé sous forme d'anneau interrompu dans sa forme annulaire, et donc ouvert, **caractérisé en ce que**
• la forme extérieure de l'anneau est conçue de manière qu'après l'implantation les extrémités ouvertes se touchent dans une zone qui soit optiquement accessible de l'extérieur de l'oeil,
• dans la zone de contact, la couleur du matériau est choisie de telle sorte qu'elle absorbe la lumière d'un laser à ondes courtes que laissent passer les milieux réfractifs de l'oeil
• et que, dans la zone de contact, la forme superficielle des parties juxtaposées est conçue de telle manière que lors du réchauffement avec un laser à ondes courtes d'une zone circonscrite située aux deux extrémités, il peut se former des zones de fusion qui se confondent.

2. Anneau de tension capsulaire selon la revendication 1, **caractérisé en ce que** ses deux extrémités s'élargissent vers le centre de l'anneau en forme d'éventail si bien qu'après implantation ces deux éventails sont placés l'un sur l'autre, où, par rapport à la direction de la cornée, la face arrière de l'éventail avant et au moins la face avant de l'éventail arrière présentent des alésages en forme de cratère, l'éventail avant étant sinon transparent.

3. Anneau de tension capsulaire selon la revendication 1, **caractérisé en ce que** ses deux extrémités s'élargissent vers le centre de l'anneau en forme d'éventail si bien qu'après implantation ces deux éventails sont placés l'un sur l'autre, où, par rapport à la direction de la cornée, l'éventail avant présente des alésages en forme de cratère qui vont en s'amenuisant vers l'éventail arrière.

4. Anneau de tension capsulaire selon la revendication 1, **caractérisé en ce que** ses deux extrémités s'élargissent vers le centre de l'anneau en forme d'éventail si bien qu'après implantation ces deux éventails sont placés l'un sur l'autre, où, par rapport à la direction de la cornée, l'éventail avant dépasse moins en direction du centre de l'anneau que l'éventail arrière et est biseauté dans la zone de contact avec l'éventail arrière.
